# DEMANDE DE BREVET EUROPEEN

(11) **EP 1 935 412 A2**
(43) Date de publication de la demande: **25.06.2008**
(21) Numéro de dépôt: 07123148.4
(22) Date de dépôt: 13.12.2007
(51) Int. Cl.: A61K 9/20, A61K 31/445

(54) **Composition pharmaceutique contenant de la 5-chloro-1-[1-[3-(2-oxo-2,3-dihydro-1H-benzimidazol-1-yl)propyl]pipéridin-4-yl]-1,3-dihydro-2H-benzimidazol-2-one et son procede de preparation**

(30) Priorité: 13.12.2006 FR 0655492
(71) Demandeur: GALENIX DEVELOPPEMENT, 33127 Saint-Jean d'Illac (FR)
(72) Inventeur: Besse, Jérôme, 33480, LISTRAC MEDOC (FR)
(74) Mandataire: Ilgart, Jean-Christophe

(57) **Abrégé**

La présente invention concerne une composition pharmaceutique, sous forme de comprimé se délitant en moins de trois minutes, comprenant de 1% à 60% en poids du principe actif dompéridone ou d'un de ses sels pharmaceutiquement acceptables ; de 20% à 90% en poids d'au moins un agent diluant sans effet notoire ; de 0,01% à 5% d'au moins un agent mouillant ; de 0,1% à 15% en poids d'au moins un agent désintégrant ; les pourcentages en poids étant exprimés par rapport au poids total de ladite composition. La présente invention concerne également l'utilisation de ladite composition pharmaceutique pour la préparation d'un médicament destiné à traiter et/ou à prévenir les nausées, les vomissements, les états nauséeux et/ou les troubles de la digestion et un procédé de préparation d'un comprimé orodispersible à base de ladite composition pharmaceutique.

## Description

### DOMAINE TECHNIQUE

La présente invention se rapporte au domaine des compositions pharmaceutiques et de leur procédé de préparation. Plus particulièrement, la présente invention concerne la fabrication d'une nouvelle composition pharmaceutique contenant le principe actif 5-chloro-1-[1-[3-(2-oxo-2,3-dihydro-1*H-*benzimidazol-1-yl)propyl]pipéridin-4-yl]-1,3-dihydro-2H-benzimidazol-2-one ou l'un de ses sels d'addition pharmaceutiquement acceptables, éventuellement sous la forme d'une association avec un second principe actif. Ladite composition pharmaceutique se présente sous la forme d'un comprimé orodispersible destiné à être administré dans la cavité buccale ou sous la langue.

La 5-chloro-1-[1-[3-(2-oxo-2,3-dihydro-1*H-*benzimidazol-1-yl)propyl]pipéridin-4-yl]-1,3-dihydro-2H-benzimidazol-2-one, dont la dénomination commune internationale (DCI) est dompéridone, est un principe actif antiémétique stimulant la motricité digestive. Sa formule développée est présentée ci-après :

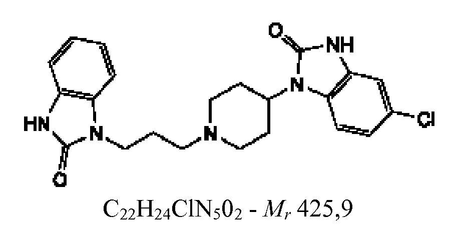

La 5-chloro-1-[1-[3-(2-oxo-2,3-dihydro-1*H-*benzimidazol-1-yl)propyl]pipéridin-4-yl]-1,3-dihydro-2H-benzimidazol-2-one se présente sous forme d'une poudre blanche ou sensiblement blanche, pratiquement insoluble dans l'eau et d'un goût désagréable. De plus, la dompéridone est un principe actif ayant une faible biodisponibilité (~15%) due à un fort effet de 1^{er} passage hépatique et intestinal.

La composition objet de l'invention exempte d'excipient à effet notoire garantit une faisabilité industrielle à l'aide d'un procédé de préparation simple et peu coûteux, une sécurité d'utilisation, une bonne stabilité à partir d'une substance active insoluble dans l'eau, ayant une granulométrie inférieure à 50 µm et sans écoulement.

### ETAT DE LA TECHNIQUE ANTERIEURE

Pour une meilleure compliance du patient, diverses compositions à libération immédiate lorsqu'administrées par voie buccale et contenant comme principe actif de la dompéridone ont été mises sur le marché.

Ainsi, le Motylio, un lyophilisat oral commercialisé par Janssen-Cilag présente l'intérêt de se déliter rapidement en dépit de la mauvaise solubilité de la substance active mais le procédé de fabrication d'une telle forme nécessitant une lyophilisation est complexe et coûteux. Par ailleurs, une fois désagrégés, les grains dispersés dans la cavité buccale confèrent une sensation granuleuse et farineuse particulièrement inconfortable.

Pour éviter l'étape de lyophilisation, il a été proposé de réaliser des comprimés dispersibles. La demande de brevet US 2006051414 décrit un comprimé dispersible dans l'eau contenant de la dompéridone ou un de ses sels pharmaceutiquement acceptables, environ 60 à 80% en poids d'un granulat auxiliaire, environ 10 à 30% d'un agent désintégrant tel que de la cellulose microcristalline et des agents édulcorant, aromatisant et lubrifiant, les % en poids étant exprimés par rapport au poids total final du comprimé. Le granulat auxiliaire est, de manière préférée, constitué de granules de D-mannitol contenant 3% d'amidon de maïs obtenues via un procédé de granulation humide.

Un autre moyen d'éviter une étape de lyophilisation est l'utilisation de comprimés ou granules effervescents. Cette forme pharmaceutique implique une fabrication en atmosphère contrôlée et, de ce fait, présente un prix de revient élevé. Par ailleurs, il a été mis en évidence que la présence de bicarbonate de sodium dans ce type de formulation réduisait la biodisponibilité de la dompéridone.

Les formes dispersibles décrites sont une excellente alternative aux suspensions buvables dont la conservation est limitée mais n'améliorent pas significativement la compliance du patient. Compte tenu de la pathologie à traiter, l'absorption de liquide pose problème. De plus, les procédés de fabrication de telles formes nécessitent, pour la plupart, une étape de granulation.

Afin d'éviter l'absorption de liquide, la demande de brevet FR2791569 décrit un comprimé dispersible et/ou orodispersible ayant les avantages du lyophilisat oral mais sans les inconvénients connus de l'étape de lyophilisation et de cette forme. Le problème de délitement lié à la faible solubilité du principe actif est résolu par la formation d'une structure expansée microporeuse isotrope à base de polymères notamment d'origine végétale. Le comprimé après désagrégation dans la cavité buccale, qui est toute aussi immédiate qu'avec un lyophilisat oral, n'est pas perceptible. Cependant, le procédé de préparation d'un tel comprimé nécessite de nombreuses étapes telles que mélange, extrusion ou injection dans un blister, puis une étape de séchage-formage micro-ondes sous vide. Le séchage micro-onde est une opération délicate, très peu utilisée dans l'industrie pharmaceutique.

Le brevet FR2845914 décrit un comprimé orodispersible dont la désagrégation rapide est réalisée en associant deux agents désintégrants/liants et en utilisant un diluant, de préférence, soluble dans l'eau de type polyol ou lactose. L'inconvénient de la composition revendiquée en dépit d'un procédé simple est la présence de diluants solubles connus pour être des excipients à effet notoire.

La demande de brevet CN1449757 décrit un comprimé orodispersible obtenu en ajoutant un agent désintégrant directement dans la composition.

Afin d'améliorer les propriétés physico-chimiques de la dompéridone et faciliter ainsi sa mise en forme pharmaceutique, la publication de Prabakaran L et Al. (Curr. Drug. Deliv. 2006 jul; 3(3):307-13) décrit une nouvelle technique de "micropelletization" mais l'usage de solvants organiques est requis.

Pour réaliser des comprimés orodispersibles à base de dompéridone sans étape de lyophilisation, l'art antérieur utilise des procédés de fabrication plus ou moins complexes, mais les plus simples tels qu'une étape de compression directe impliquent l'utilisation de diluants à effet notoire facilitant la dissolution du comprimé et conférant une sensation agréable en bouche.

Il existe donc un besoin, dans l'état de la technique, pour des formulations galéniques à base de dompéridone qui permettent de résoudre les inconvénients techniques associés aux formulations connues.

### EXPOSÉ DE L'INVENTION

Ainsi, les travaux de la Demanderesse ont permis de mettre au point une composition pharmaceutique orodispersible à base de dompéridone sans excipient à effet notoire, sans agent liant, augmentant la solubilité de la dompéridone et agréable en bouche et son procédé de fabrication par compression directe.

Un comprimé orodispersible est un comprimé non enrobé destiné à être placé dans la bouche où il se disperse rapidement avant d'être avalé (Pharmacopée Européenne, 07/2006:0478). Le temps de désagrégation de tels comprimés doit être de moins de 3 min. Cependant, afin de se rapprocher le plus possible des caractéristiques d'un lyophilisat oral, l'objectif pour le temps de désagrégation de la composition pharmaceutique selon l'invention est de 10 à 20 s. Comme détaillé ci-après, les comprimés selon l'invention ne comprennent ni enrobage, ni pelliculage.

L'invention a pour objet une composition pharmaceutique de préférence solide, orodispersible à libération immédiate, sous forme de comprimé se délitant en moins de trois minutes, contenant le principe actif dompéridone ou l'un de ses sels, caractérisée en ce qu'elle comprend :
a) de 1% à 60% en poids du principe actif dompéridone, ou d'un de ses sels pharmaceutiquement acceptables ;
b) de 20% à 90% en poids d'au moins un agent diluant sans effet notoire ;
c) de 0,01% à 5% d'au moins un agent mouillant ;
d) de 0,1% à 15% en poids d'au moins un agent désintégrant ;
les pourcentages en poids étant exprimés par rapport au poids total de ladite composition.

De préférence, la dompéridone ou l'un de ses sels pharmaceutiquement acceptables se présente sous la forme de particules solides ayant une granulométrie inférieure à 50 µm, notamment inférieure à 40 µm, encore inférieure à 30 µm, en particulier, inférieure à 20 µm et, tout particulièrement, inférieure à 10 µm. L'utilisation de dompéridone ayant une granulométrie inférieure à 50 µm augmente d'autant plus la mouillabilité de la poudre en milieu aqueux et évite la sensation granuleuse en bouche. Par « granulométrie » d'une poudre selon l'invention, on entend la taille moyenne des grains qui la constituent. Cette dernière peut être mesurée par toute technique conventionnelle connue en soi. Notamment, l'homme du métier peut avoir recours à une mesure de la granulométrie à laser du type Beckman Coulter® ou Malvern®, comme cela est décrit dans les exemples.

Des exemples de sels d'addition pharmaceutiquement acceptables utilisables dans le cadre de la présente invention comprennent, sans limitation, les sels d'addition d'acides organiques et inorganiques, non toxiques tels que le chlorhydrate, le bromhydrate, le nitrate, le perchlorate, le phosphate, le sulfate, le formiate, l'acétate, l'aconate, l'ascorbate, le benzènesulfonate, le benzoate, le bicarbonate, le bitartrate, le bisulfate, le borate, le carbonate, le cinnamate, le citrate, l'édétate, l'édétate calcique, l'embonate, l'énantate, le fumarate, le glutamate, le glycolate, le lactate, le laurate, le malate, le maléate, le malonate, le mandélate, le méthanesulfonate, le méthylsulfate, le naphtalène-2-sulfonate, la palmitate, le phtalate, le salicylate, le sorbate, le stéarate, le succinate, le tannate, le tartrate, le toluène-p-sulfonate, le valérate et analogue. On peut former de tels sels au moyen de procédés bien connus et décrits dans la technique.

D'autres acides tels que l'acide oxalique, que l'on ne peut pas considérer comme étant pharmaceutiquement acceptables, peuvent toutefois être utiles dans la préparation de sels utiles, en tant qu'intermédiaires pour obtenir un composé chimique de l'invention et son sel d'addition acide pharmaceutiquement acceptable.

La quantité de dompéridone (ou d'un sel pharmaceutiquement acceptable de cette dernière) est comprise dans la gamme allant de 1 à 60% en poids, notamment de 5 à 40% en poids et, en particulier, de 10 à 30% en poids par rapport au poids total de la composition selon la présente invention.

Par « excipient à effet notoire », on entend toute substance dont la présence au sein de la composition nécessite des précautions d'emploi pour certaines catégories particulières de patients. Seront notamment exclus de la composition selon l'invention, les polyols tels que le mannitol, xylitol, sorbitol, maltitol, sirop de maltitol, l'isomalt, l'aspartam etc.... et tout autre excipient cité dans la liste des excipients à effet notoire de l'Agence Européenne des Médicaments (EMEA) et susceptibles d'engendrer des intolérances. Ainsi, sont également exclus de la composition selon l'invention l'acide borique et ses sels à une dose supérieure à 3 mg/ kg/ jour ; l'amidon de blé ; le bronopol à une dose supérieure à 0,05 % (pourcentage en poids exprimé par rapport au poids total de la composition) ; le chlorure de benzalkonium ; les composés organomercuriels ; l'éthanol à une dose supérieure à 0,05 g/ jour ; le formaldéhyde à une dose supérieure à 0,05 % (pourcentage en poids exprimé par rapport au poids total de la composition) ; le fructose ; le galactose ; le glucose ; le glycérol à une dose supérieure à 1 g/ prise ou supérieure à 3 g/24 heures ; l'huile d'arachide ; l'huile de ricin et ses dérivés ; l'huile de soja et ses dérivés ; l'huile de sésame ; le lactose ; la paraformaldéhyde à une dose supérieure à 0,5 % (pourcentage en poids exprimé par rapport au poids total de la composition) ; le polyéthylèneglycol à une dose supérieure à 2 g/ prise ou supérieure à 6 g/ jour ; la phénylalanine ; le potassium ; le saccharose ; le sodium à une dose supérieure à 200 mg/jour pour les compositions destinées aux adultes ; le sucre inverti ; les sulfites et métabisulfites ; la tartrazine et les colorants azoïques.

Par « agent diluant », on entend selon l'invention un agent utilisé pour compléter la composition pharmaceutique préparée selon le procédé, jusqu'à obtention d'un volume total prédéterminé contenant la quantité choisie de dompéridone ou de l'un ses sels. L'agent diluant possède des propriétés liantes suffisantes dispensant de l'usage d'agent(s) liant(s) tel(s) que la povidone (ou polyvinylpyrrolidone ou PVP), les dérivés d'amidon, le polyvinyle alcool ou tout autre agent liant connu par l'homme du métier. Avantageusement, la composition pharmaceutique selon l'invention est exempte de tels agents liants.

De préférence, l'agent diluant représente de 20% à 90% en poids, notamment de 30 à 80% en poids, particulièrement de 40 à 75% en poids, mieux de 50 à 70% en poids par rapport au poids total de la composition.

Les agents diluants utilisés sont avantageusement choisis parmi la poudre de cellulose, la cellulose microcristalline, la cellulose microcristalline silicifiée, l'acétate de cellulose, le phosphate de calcium di- ou tribasique, le sulfate de calcium, les dextrates, les dextrines, les huiles végétales hydrogénées, le glyceryl palmitostéarate, le polyméthacrylate et les mélanges de ceux-ci. En effet, ces agents peuvent être utilisés seuls ou en association de deux, trois, quatre ou plus. La cellulose microcristalline est préférentiellement choisie.

Par « agent mouillant » on entend, dans le cadre de la présente invention, toute substance, sous forme solide ou liquide, modifiant la tension superficielle de l'eau sur la substance active afin d'augmenter la mouillabilité de ladite substance active en milieu aqueux. Les agents mouillants sont également connus sous le nom de surfactants ou tensioactifs.

L'agent mouillant représente de 0,01 à 5% en poids, notamment de 0,05 à 2% en poids et, en particulier, de 0,1 à 1% en poids par rapport au poids total de la composition.

L'agent mouillant est choisi parmi le dodecylsulfate, le laurilsulfate de sodium (SLS), un ester de polysorbitanne polyoxyéthyléné, tel que les esters monooléate, monolaurate, monopalmitate, monostéarate, le dioctylsulfosuccinate de sodium (DOSS), la lécithine et les mélanges de ceux-ci. En effet, ces agents peuvent être utilisés seuls ou en association de deux, trois ou quatre ou plus. Le laurilsulfate de sodium est préférentiellement choisi.

De plus, l'utilisation d'un agent désintégrant ou d'un mélange d'agents désintégrants permet de contribuer de manière essentielle aux caractéristiques mécaniques de désintégration en milieu aqueux des comprimés fabriqués.

L'agent désintégrant (ou le mélange d'agents désintégrants) représente de 0,1 à 15% en poids, notamment de 1 à 15% en poids par rapport au poids total de la composition.

L'agent désintégrant est choisi parmi la crospovidone (ou polyvinylpyrrolidone réticulée), la croscarmellose (ou carboxyméthylcellulose sodique réticulée), le glycolate d'amidon sodique, le sodium alginate, l'hydroxypropyl cellulose, l'amidon et dérivés.

La composition pharmaceutique selon l'invention peut comprendre en outre un agent édulcorant ou un mélange d'agents édulcorants, ce qui est de nature à considérablement améliorer la compliance de cette composition, compte tenu du goût désagréable du principe actif dompéridone.

La composition pharmaceutique selon l'invention peut comprendre un mélange de deux, trois ou quatre agents édulcorants, dès lors que le pourcentage en poids de l'agent édulcorant ou du mélange d'agents édulcorants va de 0,005 à 10% en poids, notamment de 0,05 à 5% en poids et, en particulier, de 0,5 à 1% en poids par rapport au poids total de la composition.

Le ou les agent(s) édulcorant(s) est(sont) avantageusement choisi(s) parmi les édulcorants de synthèse et, plus particulièrement parmi le gluconate, l'aspartame, l'acésulfame de potassium, le saccharinate de sodium, le xylitol, le maltitol et leurs mélanges. En effet, dans le cadre de la présente invention, on préfère les édulcorants de synthèse non métabolisés dans l'organisme aux édulcorants naturels et ce, pour éviter d'ajouter un excipient à effet notoire dans la composition selon l'invention.

Afin de compléter l'effet organoleptique de l'agent (ou des agents) édulcorant(s), la composition pharmaceutique selon l'invention peut aussi comprendre un agent aromatisant ou un mélange d'agents aromatisants. Ainsi, selon un mode de réalisation préférentiel de la composition pharmaceutique selon l'invention, les caractéristiques organoleptiques sont encore améliorées par l'addition de cet (ou ces) agent(s) aromatisant(s).

Ainsi, la composition pharmaceutique selon l'invention est caractérisée en ce qu'elle peut comprendre aussi de 0,005 à 10% en poids, notamment de 0,05 à 8% en poids, en particulier de 0,5 à 6% en poids et, plus particulièrement de 1 à 4% en poids d'un agent aromatisant ou d'un mélange d'agents aromatisants, par rapport au poids total de la composition.

De préférence, le ou les agent(s) aromatisant(s) est (sont) choisi(s) parmi les arômes naturels, les arômes identiques natures ou les arômes artificiels. Notamment, le ou les agent(s) aromatisant(s) est(sont) choisi(s) parmi un arôme de fruit, un arôme menthe, un arôme anis, un arôme miel, un arôme vanille, un arôme thé et un arôme verveine. Plus particulièrement, sont utilisés dans le cadre de la présente invention le ou les agent(s) aromatisant(s) suivant(s) : abricot, abricot-orange, agrumes, ananas-noix de coco, anis, banane, cacao, caramel, caramel-fruit, cassis, cerise, cerise griotte, cerise-framboise, citron, citron-vert, essence d'orange, fleur d'oranger, fraise, framboise, fruit de la passion, fruits de la forêt, fruits du verger, fruits rouges, fruits rouges/caramel, grenadine, groseille, jus d'orange, mandarine, mangue, menthe, menthe poivrée, menthe-eucalyptus, miel, mirabelle, mûre, myrtille, pamplemousse, pêche, poire, pomme, prune, pulpe d'orange, raisin, réglisse, romarin-oranger, thé, vanille, verveine ou violette.

Avantageusement, l'agent aromatisant (ou le mélange d'agents aromatisants) est adsorbé sur un support approprié avant d'être incorporé dans la composition pharmaceutique selon l'invention, sous la forme d'une poudre du support préalablement imprégné. Tout type de support conventionnel en pharmacie peut être utilisé pour l'agent aromatisant, comme par exemple une poudre de silice, d'amidon ou de cellulose.

En outre, la composition peut comprendre un agent d'écoulement ou un mélange de ceux-ci garantissant une répartition homogène de la dompéridone de faible granulométrie au sein de la composition pharmaceutique de l'invention et un bon écoulement du mélange final.

L'agent d'écoulement ou le mélange d'agents d'écoulement représente de 0,01 à 1% en poids, notamment de 0,05 à 0,8% en poids et, en particulier, de 0,1 à 0,5% en poids par rapport au poids total de la composition.

Parmi les agents d'écoulement utilisables dans le cadre de la présente invention, on peut citer la silice colloïdale, le dioxyde de silice, le silicate de magnésium ou de calcium et le talc. Dans le cadre de la présente invention, la silice colloïdale est préférentiellement choisie.

Enfin, la composition selon l'invention peut comprendre en outre un agent lubrifiant ou un mélange de ceux-ci. Au moment de la fabrication du comprimé, on ajoute une quantité appropriée d'un agent lubrifiant ou d'un mélange d'agents lubrifiants à la couche externe de ce dernier.

De préférence, la quantité appropriée de l'agent lubrifiant va de 0,01% à 2% en poids, notamment de 0,05 à 2% en poids et, en particulier, de 0,1 à 1,5% en poids par rapport au total de la composition.

Le ou les agent (s) lubrifiant (s) est (sont), de préférence, choisi(s) parmi le stéarate de magnésium, l'acide stéarique et ses dérivés, le stéarate de calcium, le stéarate de sodium, le stéaryl fumarate de sodium, l'acétate de sodium, l'oléate de sodium, le chlorate de sodium, le benzoate de sodium, le glycéril béhénate, le polyéthylène glycol, le talc et les huiles végétales hydrogénées. Le benzoate de sodium peut être utilisé aussi comme agent conservateur et anti-bactérien.

La composition pharmaceutique selon l'invention peut également comprendre d'autres excipients tels que des colorants, des agents de salivation, des conservateurs, des modificateurs de pH, etc.... L'homme du métier sait quel type de composés utiliser pour chacune des classes d'excipients ci-dessus et dans quelle proportion, la seule contrainte étant de choisir des excipients sans effet notoire. De plus, dans le cadre de la présente invention, la composition pharmaceutique peut contenir, en plus de la dompéridone, un autre (ou d'autres) principe(s) actif(s) dont l'activité thérapeutique peut être identique, comparable, complémentaire et/ou différente à celle de la dompéridone.

De préférence, la composition pharmaceutique selon l'invention est particulièrement adaptée à une administration par voie orale et, tout particulièrement, adaptée à une utilisation buccale et sublinguale ambulatoire. Ainsi, la composition pharmaceutique solide selon l'invention se présente, avantageusement, en forme posologiques unitaires contenant des quantités appropriées de dompéridone ou d'un de ses sels pharmaceutiquement acceptables. De telles quantités s'étendent de 1 à 100 mg, notamment de 2 à 50 mg et, en particulier, de 4 à 30 mg de dompéridone ou d'un de ses sels pharmaceutiques par forme posologique unitaire. La forme posologique unitaire préférée dans le cadre de la présente invention est une préparation conditionnée sous forme de comprimés et, plus particulièrement, de comprimés orodispersibles. De plus, la forme posologique unitaire peut être soit un tel comprimé en soi, soit un nombre approprié de tels comprimés. De telles formes posologiques unitaires sont particulièrement adaptées pour une ou plusieurs prise(s) quotidienne(s) en fonction de la thérapie, de la phase de la thérapie ou autre.

De part la composition qualitative et quantitative en principe actif et en excipients décrite dans l'invention, une formulation finale sous forme de comprimés peut être aisément préparée par un simple procédé de compression directe du mélange du principe actif et des excipients, dont les caractéristiques pharmacotechniques sont optimales. De préférence, un comprimé conforme à l'invention a une résistance à la rupture supérieure comprise entre 10 et 150N, en particulier entre 20 et 100N et, tout particulièrement, entre 30 et 60N et se disperse dans l'eau distillée à 20°C en moins de 3 minutes, mieux en moins de 1,5 minute et encore mieux en moins de 1 minute.

La présente invention concerne également l'utilisation d'une composition pharmaceutique telle que précédemment définie pour la préparation d'un médicament destiné à traiter et/ou à prévenir les nausées, les vomissements, les états nauséeux et/ou les troubles de la digestion.

La présente invention concerne aussi une méthode de traitement curatif et/ou préventif des nausées, des vomissements, des états nauséeux et/ou des troubles de la digestion, ladite méthode consistant à administrer à un patient une quantité thérapeutiquement efficace d'une composition pharmaceutique telle que précédemment définie. Dans le cadre de la présente invention, on entend par « patient » un homme ou une femme, de tout âge i.e. du nouveau-né à la personne âgée, en bonne santé, souffrant ou susceptible de souffrir de nausées, de vomissements, d'un état nauséeux et/ou de troubles de la digestion.

Enfin, la présente invention a pour objet un procédé pour préparer un comprimé orodispersible contenant comme principe actif de la dompéridone, caractérisé en ce qu'il comprend les étapes consistant à :
1) mélanger à homogénéité la dompéridone ou un de ses sels pharmaceutiquement acceptable, au moins un agent diluant sans effet notoire, au moins un agent mouillant, au moins un agent désintégrant tels que précédemment définis et dans les proportions précédemment définies et, éventuellement, un ou plusieurs autre(s) excipient (s) et/ou principe(s) actif(s),
2) éventuellement tamiser le mélange et/ou en cours de préparation dudit mélange et ce, pour enlever les agglomérats non désirés,
3) éventuellement lubrifier ledit mélange en ajoutant un agent de lubrification tel que précédemment défini et dans les proportions précédemment définies,
4) compresser ledit mélange ayant éventuellement été tamisé et/ou lubrifié dans une machine à comprimer munie de poinçons adéquats pour obtenir un comprimé orodispersible délivrant la quantité désirée de dompéridone.

Les éventuels autres excipients et autres principes actifs ajoutés au mélange du procédé de l'invention comprennent notamment les excipients et principes actifs précédemment décrits et, en particulier, les agents d'écoulement, aromatisants et édulcorants tels que précédemment définis et dans les proportions précédemment définies.

Le mélange des ingrédients (agent actif et excipients) peut être réalisé en ajoutant ces ingrédients soit de façon simultanée, soit de façon séquentielle. L'ajout de façon séquentielle des ingrédients permet d'introduire une ou plusieurs étapes de tamisage et/ou de lubrification au cours de l'étape de mélange.

Dans une première forme de mise en oeuvre du procédé de préparation selon l'invention, l'étape (1) de ce dernier peut comprendre les étapes consistant à :
a) mélanger la dompéridone ou un de ses sels pharmaceutiquement acceptables dans les proportions précédemment définies avec au moins un agent mouillant tel que précédemment défini et dans les proportions précédemment définies et au moins un agent d'écoulement tel que précédemment défini et dans les proportions précédemment définies,
b) éventuellement, tamiser le mélange de l'étape (a),
c) ajouter au moins un agent diluant tel que précédemment défini puis mélanger,
d) éventuellement, tamiser le mélange de l'étape (c),
e) ajouter au mélange éventuellement tamisé de l'étape (d), au moins un agent diluant tel que précédemment défini et au moins un agent désintégrant, au moins un agent aromatisant et au moins un agent édulcorant tels que précédemment définis et dans les proportions définies puis mélanger,
   les agents diluants des étapes (c) et (e) identiques ou différents étant tels que la proportion finale d'agent(s) diluant(s) dans le comprimé final est de 20% à 90% en poids par rapport au poids total du comprimé.

Dans une seconde forme de mise en oeuvre du procédé de préparation selon l'invention, l'étape (1) de ce dernier peut comprendre les étapes consistant à :
a') mélanger de la dompéridone ou un de ses sels pharmaceutiquement acceptables avec au moins un agent mouillant tel que précédemment défini et dans les proportions précédemment définies et au moins un agent d'écoulement tel que précédemment défini et dans les proportions précédemment définies,
b') éventuellement, tamiser le mélange de l'étape (a'),
c') ajouter au moins un agent diluant tel que précédemment défini et de la dompéridone ou un de ses sels pharmaceutiquement acceptables puis mélanger,
d') éventuellement, tamiser le mélange de l'étape (c'),
e') ajouter au mélange éventuellement tamisé de l'étape (d'), au moins un agent diluant tel que précédemment défini
f') éventuellement, tamiser le mélange de l'étape (e'),
g') ajouter au mélange éventuellement tamisé de l'étape (f'), au moins un agent diluant tel que précédemment défini et au moins un agent désintégrant, au moins un agent aromatisant et au moins un agent édulcorant tels que précédemment définis et dans les proportions définies puis mélanger,
les agents diluants des étapes (c'), (e') et (g') identiques ou différents étant tels que la proportion finale d'agent(s) diluant(s) dans le comprimé final est de 20% à 90% en poids par rapport au poids total du comprimé,
et la quantité de dompéridone ou d'un de ses sels pharmaceutiquement acceptables étant telle que leur proportion finale dans le comprimé final est de 1% à 60% en poids par rapport au poids total du comprimé.

Le mélange obtenu en fin d'étapes (e) ou (g') est soumis aux étapes 2, 3 et 4 du procédé telles que précédemment définies. Les différents ingrédients ajoutés aux étapes (c), (e), (c'), (e') et (g') des procédés peuvent être soumis à un tamisage avant cet ajoût.

Le choix des poinçons de la machine à comprimer, de leur forme et de leur taille, des conditions opératoires lors des éventuels tamisages ou de la compression est un travail de routine pour l'homme du métier.

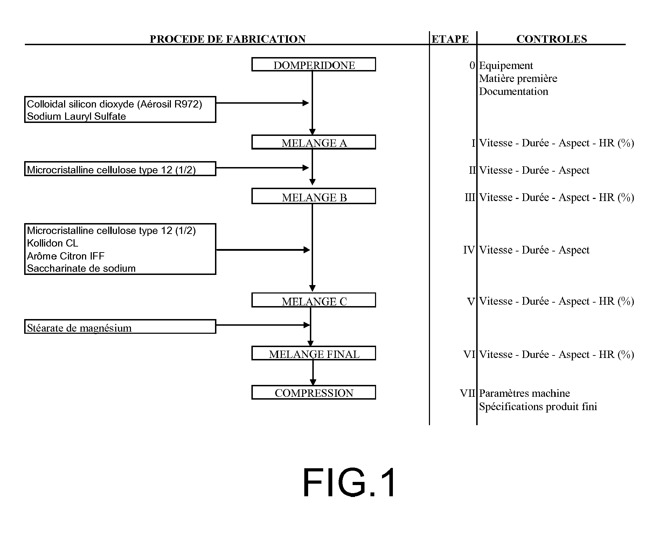

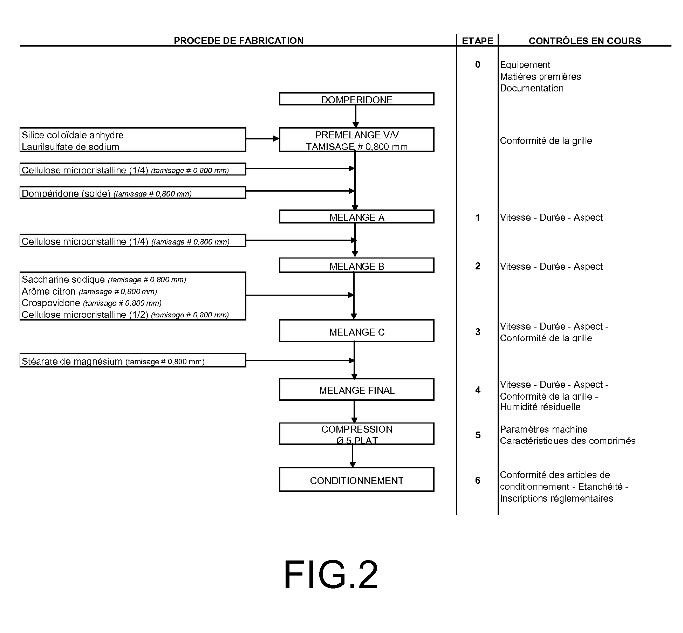

Ainsi, dans la première forme de mise en oeuvre du procédé de préparation selon l'invention (Schéma de fabrication 1, Figure 1), le comprimé est obtenu par simple dilution du principe actif avec les différents excipients présents dans la composition, lubrification puis compression directe, alors que, dans la seconde forme de mise en oeuvre (Schéma de fabrication 2, Figure 2), le comprimé est obtenu par double dilution du principe actif avec les différents excipients présents dans la composition, lubrification puis compression directe. La double dilution améliore très significativement l'homogénéité du mélange final et les résultats d'uniformité de teneur des comprimés. Le mode de réalisation comprenant une étape de double dilution est préférentiellement choisi.

De manière surprenante, il a été mis en évidence que la composition qualitative et quantitative et son procédé de fabrication selon l'invention, permettent d'obtenir un profil de dissolution *in vitro* et des profils pharmacocinétiques *in vivo* comparables à un lyophilisat oral à base de dompéridone commercialisée.

Ainsi, grâce à la composition pharmaceutique solide et son procédé de fabrication selon l'invention, on peut préparer des comprimés orodispersibles qui possèdent un profil de dissolution *in vitro* de la dompéridone dans une solution tampon à pH 1,2 avec plus de 75% de substance active libérée en moins de 5 min.

On peut également préparer des comprimés orodispersibles de dompéridone qui possèdent un profil pharmacocinétique *in vivo* suivant :
- de préférence, le comprimé orodispersible de dompéridone de l'invention possède un profil pharmacocinétique *in vivo* caractérisé par une aire sous la courbe de concentration plasmatique de 0 itio à l'infini (AUC0-∞), comprise entre 20 et 85 ng.h/ml ;
- de préférence, le comprimé orodispersible de dompéridone de l'invention possède un profil pharmacocinétique *in vivo* caractérisé par une valeur de concentration plasmatique maximum (Cmax) comprise entre 5 et 40 ng/ml) ;
- de préférence, le comprimé orodispersible de dompéridone de l'invention possède un profil pharmacocinétique *in vivo* caractérisé par une valeur de Tmax comprise entre 0,1 et 3h. Par « valeur de Tmax », l'homme du métier comprendra le temps nécessaire à l'obtention de la concentration plasmatique maximum du principe actif ;
- de préférence, le comprimé orodispersible de dompéridone de l'invention possède un profil pharmacocinétique *in vivo* caractérisé par une demi-vie plasmatique (t_{1/2}) comprise entre 5 et 10h.

D'autres caractéristiques et avantages de la présente invention apparaîtront encore à la lecture des exemples ci-après donnés à titre illustratif et non limitatif et faisant référence aux figures annexées.

### BRÈVE DESCRIPTION DES DESSINS

La figure 1 présente le schéma de fabrication 1 mis en oeuvre pour les formulations GAL345.03 et GAL345.04 et conforme à la première variante du procédé de fabrication tel que précédemment défini ;
La figure 2 présente le schéma de fabrication 2 utilisé pour la préparation de la formulation GAL345.05 et conforme à la deuxième variante du procédé de fabrication tel que précédemment défini ;
La figure 3 présente la comparaison des profils de dissolution *in vitro* obtenus pour 3 formulations selon l'invention (GAL345.03, GAL345.04 et GAL345.05) et pour un lyophilisat oral à base de dompéridone commercialisée (produit de référence).

### Exemple 1 : Compositions orodispersibles de dompéridone 10 mg obtenues par un procédé de compression directe

### A. Choix des excipients

*Cellulose microcristalline* : Cet excipient est un agent liant couramment utilisé dans la fabrication de comprimés. Plusieurs types de cellulose microcristalline sont disponibles. Ils diffèrent par leur mode de fabrication, la taille des particules, l'humidité résiduelle, l'écoulement ou d'autres propriétés physiques. Pour le développement d'un comprimé orodispersible, le choix s'est porté sur du Vivapur® 12 commercialisé par la société JRS. En effet, celui-ci possède une humidité résiduelle inférieure à 5%, un bon écoulement, une distribution granulométrique adaptée aux autres composants (taille des particules moyenne de 180 µm) et de bonne aptitude à la compression directe. Tout approvisionnement de qualité équivalente peut se substituer à celui-ci. La cellulose microcristalline est conforme aux spécifications de la monographie (0316) de la Pharmacopée Européenne en vigueur.

*Crospovidone* : Cet excipient est un agent désintégrant généralement utilisé en mélange à sec à hauteur de 2 à 5% dans les formules de l'état de la technique. Dans la formule selon l'invention, il représente environ 12% de la masse totale d'un comprimé afin d'obtenir un effet désintégrant et répondre aux objectifs à atteindre en matière de comprimé orodispersible. La crospovidone utilisée est le Kollidon® CL commercialisé par la société BASF. Tout approvisionnement de qualité équivalente peut se substituer à celui-ci. La crospovidone est conforme aux spécifications de la monographie (0892) de la Pharmacopée Européenne en vigueur.

*Stéarate de magnésium* : Cet excipient est couramment utilisé dans les spécialités pharmaceutiques pour ses propriétés lubrifiantes. Le stéarate de magnésium d'origine végétale utilisé au cours du développement est fourni par la société FACI. Tout approvisionnement de qualité équivalente peut se substituer à celui-ci. Le stéarate de magnésium est conforme aux spécifications de la monographie (0229) de la Pharmacopée Européenne

*Saccharine sodique* : La saccharine sodique est un édulcorant intense utilisé dans les boissons, les produits alimentaires, les édulcorants de table et les formulations pharmaceutiques. La saccharine sodique est considérablement plus soluble dans l'eau que la saccharine et également plus fréquemment employée dans des formulations pharmaceutiques. Son pouvoir édulcorant est approximativement 300 fois plus puissant que le sucre. La saccharine sodique améliore les saveurs et peut être employée pour masquer certaines caractéristiques désagréables de goût. Toutes ces caractéristiques sont conformes à l'objectif de développer un comprimé orodispersible. Le laboratoire COOPER fournit la saccharine sodique utilisée pendant le développement. Tout approvisionnement de qualité équivalente peut se substituer à celui-ci. La saccharine sodique est conforme aux spécifications de la monographie (0787) de la Pharmacopée Européenne en vigueur.

*Laurilsulfate de sodium* [Ph. Eur. (0098)] : Il s'agit d'un agent mouillant utilisé dans la fabrication des comprimés lorsque la substance active est peu soluble. Il permet d'améliorer la mouillabilité de l'actif. Cet excipient est fourni par le laboratoire COOPER. Tout approvisionnement de qualité équivalente peut se substituer à celui-ci. Le laurilsulfate de sodium est conforme aux spécifications de la monographie (0098) de la Pharmacopée Européenne en vigueur.

*Silice hydrophobe colloïdale anhydre* : Cet excipient est utilisé dans la formule comme agent d'écoulement afin de permettre une bonne répartition de la substance active. Ainsi, l'utilisation de la silice colloïdale permet de répartir correctement la substance active dans le mélange. La silice colloïdale utilisée est fournie par la société DEGUSSA sous la dénomination Aerosil® R972. Cette qualité dispose de propriétés hydrophobes et permet au comprimé orodispersible d'être moins sensible au phénomène de reprise d'humidité. Tout approvisionnement de qualité équivalente peut se substituer à celui-ci. La monographie Silice hydrophobe colloïdale anhydre est conforme aux spécifications de la monographie (2208) de la Pharmacopée Européenne en vigueur.

*Arôme citron* : Une aromatisation de type agrume a été sélectionnée en vue de masquer le goût désagréable de la substance active. Cet arôme fait l'objet d'une monographie interne.

### B. Compositions et caractéristiques des formules réalisées

Le Tableau 1 ci-après reprend, pour chaque formule réalisée dans le cadre de la présente invention, sa composition et ses caractéristiques.

**Tableau 1**

| **N° de formule x** | **GAL 345.03** | **GAL 345.04** | **GAL 345.04** |
|---|---|---|---|
| Composition (%) | | | |
| Dompéridone | 18,182 | 18,182 | 18,182 |
| Arôme citron | 2,364 | 2,364 | 2,364 |
| Cellulose microcristalline type 12 | 65,364 | 65,091 | 65,091 |
| Stéarate de magnésium | 1,181 | 1,181 | 1,181 |
| Saccharine sodique | 0,582 | 0,582 | 0,582 |
| Silice hydrophobe colloïdale | 0,236 | 0,236 | 0,236 |
| anhydre | | | |
| Crospovidone | 11,818 | 11,818 | 11,818 |
| Laurilsulfate de sodium | 0;273 | 0,546 | 0,546 |

| Contrôle du mélange final | Lot 04VG022602 | Lot 04VR030501 | Lot 04CDA042801 |
|---|---|---|---|
| Humidité (%) | 2,59 | - | 4,36 |
| Ecoulement (s) Mesure du volume apparent : | 2,45 pour 50 g | | 6,65 |
| | | - | |
| avant tassement (m/V0) | 0,44 | - | 0,43 |
| après tassement (m/V1250) | - | - | 0,54 |
| après tassement (m/V2500) | 0,59 | - | - |
| aptitude au tassement (ml) | 28 | - | 30 |
| Analyse granulométrique (centrage en µm) | - | - | 125 |
| Caractères | - | - | Mélange homogène |

| Contrôle du produit fini | Lot 04VG022604 | Lot 04CDA030501 | Lot 04CDA050301 |
|---|---|---|---|
| Format de poinçons | 5 plat | 5 plat | 5 plat |
| Humidité (%) | - | - | - |
| Masse moyenne (mg) | 54,61 | 54,49 | 55,60 |
| Uniformité de masse | Conforme | Conforme | Conforme |
| Résistance à la rupture (N) | 58,2 | 58,7 | 44,3 |
| Désagrégation | 30 s | 27 s | 18 s |
| Contrôles dimensionnels : | | | |
| Epaisseur (mm) | 2,23 | - | 2,75 |
| Diamètre (mm) | 5,04 | - | 5,05 |
| Friabilité (%) | 0,120 | 0,01 | < 0,01 |
| Masse théorique du comprimé (mg) | 55,00 | 55,00 | 55,00 |

### C. Mode opératoire

La figure 1 présente le schéma de fabrication 1 mis en oeuvre pour les formulations GAL345.03 et GAL345.04, alors que le schéma de fabrication 2 utilisé pour la préparation de la formulation GAL345.05 est présenté à la figure 2.

### Etape 1 : MELANGE A

- Prémélanger à volume égal la dompéridone avec la silice hydrophobe colloïdale anhydre et le laurilsulfate de sodium.
- Tamiser sur grille d'ouverture de maille 0,8 mm et incorporer dans la cuve 1/8 de la quantité de cellulose microcristalline, le prémélange et le solde de la quantité de dompéridone.
- Tamiser sur grille d'ouverture de maille 0,8 mm et ajouter 1/8 de la quantité de cellulose microcristalline ayant préalablement servi à rincer le sac contenant la dompéridone
- Mélanger à la roue Rohn pendant environ 2 min à 24 tr.min⁻¹.

### Etape 2 : MELANGE B

- Ajouter, dans la cuve contenant le mélange A, 1/4 de la quantité de cellulose microcristalline préalablement tamisée sur une grille d'ouverture de maille 0,8 mm.
- Mélanger à la roue Rohn pendant environ 2 min à 24 tr.min-1.

### Etape 3 : MELANGE C

- Ajouter, dans la cuve contenant le mélange B, la saccharine sodique, l'arôme citron, la crospovidone et la cellulose microcristalline préalablement tamisés sur une grille d'ouverture de maille 0,8 mm.
- Mélanger à la roue Rohn pendant environ 7 min à 24 tr.min-1.

### Etape 4 : MELANGE FINAL

- Ajouter, dans la cuve contenant le mélange C, le stéarate de magnésium préalablement tamisé sur une grille d'ouverture de maille 0,8 mm.
- Mélanger à la roue Rohn pendant environ 2 min à 24 tr.min-1.

### Etape 5 : COMPRESSION

- Equiper la machine à comprimer avec les poinçons adéquats (diamètre 5 rond plat)
- Procéder au réglage de la machine de manière à obtenir des comprimés conformes aux spécifications décrites en 3.2.P.3.4.
- Réaliser la compression du mélange final.

### Etape 6 : CONDITIONNEMENT

Conditionner les comprimés en blister thermoformé PVC/PVDC/Aluminium.

### D. Etude de dissolution in vitro comparée

Les conditions opératoires mises en oeuvre pour cette étude sont les suivantes :

| | |
|---|---|
| Equipement : | Dissolutest Sotax AT7 - spectrophotomètre Lambda 20 |
| Méthode : | Spectrophotométrie UV selon Ph.Eur. (2.9.3) |
| Composé dosé : | dompéridone |
| Vol de la cuve: | 500 ml |
| Longueur d'onde : | 206 nm |
| Milieu : | Tampon pH 1,2 |
| Rotation de l'axe : | 75 rpm |

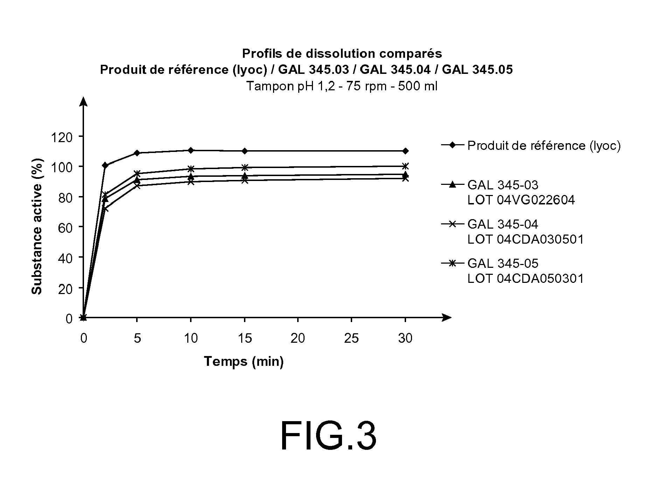

Les profils de dissolution *in vitro* obtenus pour les trois formulations selon l'invention (GAL345.03, GAL345.04 et GAL345.05) et pour un lyophilisat oral à base de dompéridone commercialisé (produit de référence) sont présentés à la figure 3.

### Exemple 2 : Etude pharmacocinétique in vivo de bioéquivalence

Dans cet exemple, ont été étudiées les caractéristiques de pharmacocinétique *in vivo* de comprimés orodispersibles de dompéridone dosés à 10 mg préparés conformément à la formulation GAL345.05 versus un lyophilisat oral à base de dompéridone dosée à 10 mg commercialisé.

### A. Méthode d'analyse des données de pharmacocinétique in vivo

Le taux et l'étendue de l'absorption de la dompéridone après administration d'une formulation de comprimés orodispersibles conforme à l'invention et dosée à 10 mg ont été évalués. La même étude a été réalisée après administration de lyophilisat oral à base de dompéridone et dosée à 10 mg afin de mettre en évidence la bioéquivalence entre les 2 formulations.

Ont été déterminées les variables pharmacocinétiques suivantes :
- la valeur d'aire sous la courbe de concentration plasmatique de 0 itio à l'infini (AUC0-∞)
- la valeur de concentration plasmatique maximum (Cmax),
- le profil pharmacocinétique *in vivo* caractérisé par la valeur de Tmax,
- le temps de demi-vie plasmatique (t1/2).

Cette étude a consisté en l'administration randomisée d'une dose unique, en cross-over, en ouvert. Elle a été réalisée sur 34 individus hommes et femmes sains, de manière à réaliser l'étude avec au moins 28 sujets susceptibles d'être évalués. L'âge des sujets était compris entre 18 et 56 ans. Les individus soumis à l'étude étaient présents au moins 11 heures avant de recevoir une dose orale unique de 10 mg de dompéridone (comprimé orodispersible ou lyophilisat oral) et sont restés pendant les 24 heures suivantes.

Pour la réalisation de l'étude, on a prélevé 19 échantillons d'un volume de 20 ml de sang veineux dans des tubes de verre héparinés, prélevés sur une durée de 36 heures. Des échantillons d'un volume de 10 ml de sang veineux ont été recueillis sur chaque individu, respectivement avant la prise orale du comprimé de la formulation de test selon l'invention, et aux temps 0.17, 0.33, 0.5, 0.67, 0.83, 1, 1.33, 1.67, 2, 2.5, 3, 4, 5, 7, 9, 1, 16, 24, 36 heures après la prise orale du comprimé.

La concentration de dompéridone, exprimée en ng/ml, a été mesurée dans chacun des échantillons de sang prélevé. Les analyses statistiques des paramètres Cmax, AUC (0-∞) et t1/2 ont inclus l'analyse de variance, après transformation logarithmique des données. On a aussi calculé des intervalles de confiance à 90 % pour chaque point de donnée.

### B. Résultats

Les données recueillies pendant l'étude montrent que les comprimés orodispersibles de dompéridone préparés selon la formulation GAL345.05 de l'exemple 1 de l'invention et le lyophilisat oral à base de dompéridone commercialisé, possèdent les caractéristiques de pharmacocinétique *in vivo* suivantes :

### Données pharmacocinétiques

**Tableau 2**

| **Lyophilisat oral commercialisé à base de dompéridone dosé à 10 mg** | | |
|---|---|---|
| **Variable** | **Géométrie moyenne *(SD)*** | **Intervalle** |
| **Cmax (ng/ml)** | 13.04 *(7.00)* | 5.46-35.67 |
| **AUC(0-tmax) (hr.ng/ml)** | 40.22 *(11.62)* | 23.37-73.99 |
| **AUC(0-∞)** (hr.ng/ml) | 45.45 *(11.99)* | 26.67-81.25 |
| **t ½ (hr)** | 8.06 | 3.71-13.14 |
| **Tmax (hr)** | 0.84 | 0.33-2.50 |

| **Comprimé orodispersible selon l'invention (Formulation GAL345.05) dosé à 10 mg** | | |
|---|---|---|
| **Variable** | **Géométrie moyenne *(SD)*** | **Intervalle** |
| **Cmax (ng/ml)** | 13.21 (6.48) | 5.47-35.55 |
| **AUC(0-tmax) (hzr.ng/ml)** | 39.69 (13.11) | 21.32-74.49 |
| **AUC (0-∞)** (hr.ng/ml) | 44.57 (13.95) | 24.53-81.94 |
| **t ½ (hr)** | 7.24 | 3.44-14.06 |
| **Tmax (hr)** | 0.89 | 0.50-2.00 |

### Comprimés orodispersibles selon l'invention versus Lyophilisat oral commercialisé

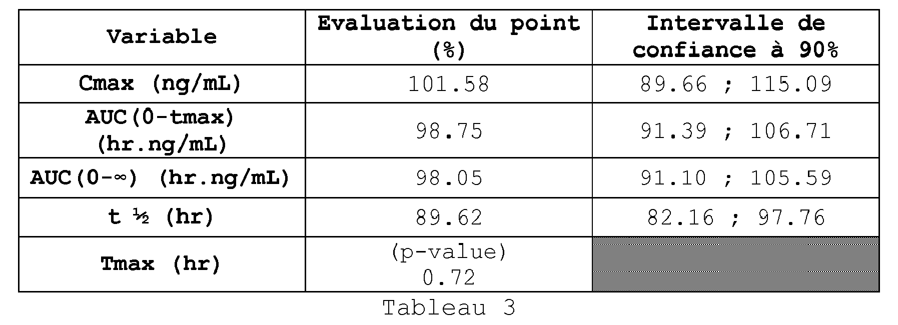

### Effets secondaires

**Tableau 4**

| | **Vertiges** | **Maux de tête** |
|---|---|---|
| **Lyophilisat oral commercialisé** | 4 | - |
| **Comprimé orodispersible selon l'invention** | 1 | 1 |

L'étude pharmacocinétique met clairement en évidence que la composition de comprimé orodispersible à base de dompéridone selon l'invention est équivalente au produit commercialisé sous forme de lyophilisat oral (Tableaux 2 et 3).

Les effets secondaires de la composition de comprimé orodispersible à base de dompéridone selon l'invention sont moins importants qu'avec le lyophilisat oral commercialisée (Tableau 4).

## Revendications

1. Composition pharmaceutique sous forme de comprimé se délitant en moins de trois minutes, contenant le principe actif dompéridone, **caractérisée en ce qu'**elle comprend :
a) de 1% à 60% en poids du principe actif dompéridone ou d'un de ses sels pharmaceutiquement acceptables ;
b) de 20% à 90% en poids d'au moins un agent diluant sans effet notoire ;
c) de 0,01% à 5% d'au moins un agent mouillant ;
d) de 0,1% à 15% en poids d'au moins un agent désintégrant ;
les pourcentages en poids étant exprimés par rapport au poids total de ladite composition.

2. Composition pharmaceutique selon la revendication 1, ledit agent diluant est choisi parmi la poudre de cellulose, la cellulose microcristalline, la cellulose microcristalline silicifiée, l'acétate de cellulose, le phosphate de calcium di- ou tribasique, le sulfate de calcium, les dextrates, les dextrines, les huiles végétales hydrogénées, le glyceryl palmitostéarate, le polyméthacrylate et les mélanges de ceux-ci.

3. Composition pharmaceutique selon l'une quelconque des revendications 1 ou 2, **caractérisée en ce que** ledit agent mouillant est choisi parmi le dodecylsulfate, le laurilsulfate de sodium (SLS), un ester de polysorbitanne polyoxyéthyléné, tel que les esters monooléate, monolaurate, monopalmitate, monostéarate, le dioctylsulfosuccinate de sodium (DOSS), la lécithine et les mélanges de ceux-ci.

4. Composition pharmaceutique selon l'une quelconque des revendications 1 à 3, **caractérisée en ce que** ledit agent désintégrant est choisi parmi la crospovidone (ou polyvinylpyrrolidone réticulée), la croscarmellose (ou carboxyméthylcellulose sodique réticulée), le glycolate d'amidon sodique, le sodium alginate, l'hydroxypropyl cellulose, l'amidon et dérivés.

5. Composition pharmaceutique selon l'une quelconque des revendications 1 à 4, **caractérisée en ce qu'**elle comprend en outre de 0,005 à 10% en poids par rapport au poids total de la composition d'un agent édulcorant ou d'un mélange d'agents édulcorants et, de préférence, d'un agent édulcorant de synthèse ou d'un mélange d'agents édulcorants de synthèse.

6. Composition pharmaceutique selon l'une quelconque des revendications 1 à 5, **caractérisée en ce qu'**elle comprend en outre de 0,005 à 10% en poids d'un agent aromatisant ou d'un mélange d'agents aromatisants, par rapport au poids total de la composition.

7. Composition pharmaceutique selon la revendication 6, **caractérisée en ce que** ledit ou lesdits agent(s) aromatisant(s) est(son)t adsorbé(s) sur un support approprié.

8. Composition pharmaceutique selon l'une quelconque des revendications 1 à 7, **caractérisée en ce qu'**elle comprend en outre de 0,01 à 1% en poids d'un agent d'écoulement ou d'un mélange d'agents d'écoulement par rapport au poids total de la composition.

9. Composition pharmaceutique selon l'une quelconque des revendications précédentes, **caractérisée en ce qu'**elle comprend en outre de 0,01% à 2% en poids d'un agent lubrifiant ou d'un mélange d'agents lubrifiants par rapport au total de la composition.

10. Composition pharmaceutique selon l'une quelconque des revendications précédentes, **caractérisée en ce qu'**elle se présente sous forme de comprimés orodispersibles.

11. Utilisation d'une composition pharmaceutique selon l'une quelconque des revendications précédentes pour la préparation d'un médicament destiné à traiter et/ou à prévenir les nausées, les vomissements, les états nauséeux et/ou les troubles de la digestion.

12. Procédé de préparation d'un comprimé orodispersible, **caractérisé en ce qu'**il comprend les étapes consistant à :
1) mélanger à homogénéité la dompéridone ou un de ses sels pharmaceutiquement acceptable, au moins un agent diluant sans effet notoire, au moins un agent mouillant, au moins un agent désintégrant tels que définis dans les revendications 1 à 4, eventuellement, un ou plusieurs autre(s) excipient(s) et/ou principe(s) actif(s),
2) éventuellement tamiser le mélange et/ou en cours de préparation dudit mélange,
3) éventuellement lubrifier ledit mélange en ajoutant un agent de lubrification tel que défini dans la revendication 10,
4) compresser ledit mélange ayant éventuellement été tamisé et/ou lubrifié dans une machine à comprimer munie de poinçons adéquats pour obtenir un comprimé orodispersible délivrant la quantité désirée de dompéridone.

13. Procédé selon la revendication 12, **caractérisé en ce que** ladite étape (1) comprend les étapes consistant à :
a) mélanger la dompéridone ou un de ses sels pharmaceutiquement acceptables dans les proportions définies à la revendication 1 avec au moins un agent mouillant tel que défini dans les revendications 1 et 3 et au moins un agent d'écoulement tel que défini dans la revendication 8,
b) éventuellement, tamiser le mélange de l'étape (a),
c) ajouter au moins un agent diluant tel que défini dans les revendications 1 et 2 puis mélanger,
d) éventuellement, tamiser le mélange de l'étape (c),
e) ajouter au mélange éventuellement tamisé de l'étape (d), au moins un agent diluant tel que défini dans les revendications 1 et 2 et au moins un agent désintégrant tel que défini dans les revendications 1 et 4, au moins un agent aromatisant tel que défini dans les revendications 6 et 7, et au moins un agent édulcorant tel que défini dans la revendication 5 puis mélanger,
les agents diluants des étapes (c) et (e) identiques ou différents étant tels que la proportion finale d'agent(s) diluant(s) dans le comprimé final est de 20% à 90% en poids par rapport au poids total du comprimé.

14. Procédé selon la revendication 12, **caractérisé en ce que** ladite étape (1) comprend les étapes consistant à :
a') mélanger de la dompéridone ou un de ses sels pharmaceutiquement acceptables avec au moins un agent mouillant tel que défini dans les revendications 1 et 3 et au moins un agent d'écoulement tel que défini dans la revendication 8,
b') éventuellement, tamiser le mélange de l'étape (a'),
c') ajouter au moins un agent diluant tel que défini dans les revendications 1 et 2 et de la dompéridone ou un de ses sels pharmaceutiquement acceptables puis mélanger,
d') éventuellement, tamiser le mélange de l'étape (c'),
e') ajouter au mélange éventuellement tamisé de l'étape (d'), au moins un agent diluant tel que défini dans les revendications 1 et 2,
f') éventuellement, tamiser le mélange de l'étape (e'),
g') ajouter au mélange éventuellement tamisé de l'étape (f'), au moins un agent diluant tel que défini dans les revendications 1 et 2 et au moins un agent désintégrant tel que défini dans les revendications 1 et 4, au moins un agent aromatisant tel que défini dans les revendications 6 et 7, et au moins un agent édulcorant tel que défini dans la revendication 5 puis mélanger,
les agents diluants des étapes (c'), (e') et (g') identiques ou différents étant tels que la proportion finale d'agent(s) diluant(s) dans le comprimé final est de 20% à 90% en poids par rapport au poids total du comprimé,
et la quantité de dompéridone ou d'un de ses sels pharmaceutiquement acceptables étant telle que leur proportion finale dans le comprimé final est de 1% à 60% en poids par rapport au poids total du comprimé.
